Europäisches Patentamt

European Patent Office

Office européen des brevets

(19)

(11) Publication number: **0 397 463**

**A2**

(12) **EUROPEAN PATENT APPLICATION**

(21) Application number: 90304978.1

(22) Date of filing: 09.05.90

(51) Int. Cl.⁵: **C12N 15/10, C12N 15/48**

(30) Priority: 10.05.89 US 349666

(43) Date of publication of application:
**14.11.90 Bulletin 90/46**

(84) Designated Contracting States:
**DE FR GB**

(71) Applicant: **EASTMAN KODAK COMPANY**
**343 State Street**
**Rochester, New York 14650(US)**

(72) Inventor: **Holzschu, Donald Lee, c/o Eastman**
**Kodak Comp.**
**Patent Department, 343 State Street**
**Rochester, New York 14650(US)**

Inventor: **McCoon, Patricia Elizabeth, c/o**
**Eastman Kodak Comp**
**Patent Department, 343 State Street**
**Rochester, New York 14650(US)**
Inventor: **Ciccarelli, Richard Benjamin, c/o**
**Eastman Kodak C**
**Patent Department, 343 State Street**
**Rochester, New York 14650(US)**

(74) Representative: **Nunney, Ronald Frederick**
**Adolphe et al**
**Kodak Limited Patent Department Headstone**
**Drive**
**Harrow Middlesex HA1 4TY(GB)**

(54) **Construction of synthetic double-stranded DNA sequences.**

(57) A method of synthesizing double-stranded DNA
involving insertion of related oligonucleotides by site-
directed mutagenesis and genetic enrichments is
disclosed.

FIG. I

EP 0 397 463 A2

## CONSTRUCTION OF SYNTHETIC DOUBLE-STRANDED DNA SEQUENCES

This invention relates to the field of recombinant DNA. More particularly this invention relates to a method of constructing double-stranded DNA sequences such as genes.

The production of foreign proteins in procaryotic and eucaryotic cells involves inserting the appropriate genes into plasmids(vectors) containing the proper regulatory sequences for gene expression, followed by transfection of these expression vectors into an appropriate host cell.

The gene(s) are obtained by standard gene cloning techniques such as described in Molecular Cloning: A Laboratory Manual (1982), Maniatis et al. Such techniques involve direct isolation of the gene from chromosomes (U.S. Patent 4,707,455), or indirect isolation of the gene through an mRNA intermediate.

Alternatively, advances in DNA chemistry, instrumentation and enzymology have made in vitro synthesis of genes from specific DNA oligonucleotides possible. Synthetic genes potentially have several advantages over their cloned natural counterparts. They are usually more compact than eucaryotic genes since they contain no intron regions within the reading frame. Also, they do not contain large DNA flanking sequences which are commonly present when genes are isolated. In addition, due to the degeneracy in the genetic code, amino acid codons can be chosen to match the optimal codons utilized by the host, leading to higher levels of protein production. Unique DNA restriction enzyme sites can be part of the design of the gene, or can be added to the ends of the gene, which results in a way to clone the gene into an expression vector, and also allows for screening for the gene upon its introduction into the host. In addition, gene regulatory sequences such as promoters, enhancers, and translational control signals are conveniently designed into the gene for the purpose of controlling protein production in the host.

Various strategies for chemically synthesizing genes in vitro have been used to construct a wide variety of genes. See Nucleic Acids Research 16 (1988) pp. 4287-98, Groger et al. Most of these strategies involve making oligonucleotides comprising both DNA strands of the gene and ultimately joining them together to give the complete double-stranded gene. The most widely used strategy involves chemical synthesis of both strands in a series of short, overlapping oligonucleotides (each approximately 40 nucleotides in length) which, when annealed and joined (ligated) together, comprise the complete gene.

For example, to chemically synthesize a relatively small gene of 400 base pairs (bp), this common strategy requires that a total of 800 bases (400 x 2 strands) be synthesized. This could be accomplished by separately synthesizing 20 oligonucleotides of 40 bases each. These would then be purified by gel electrophoresis and/or high performance liquid chromatography. Following purification, these twenty individual single-stranded oligonucleotides are phosphorylated, mixed together and heated, and allowed to cool slowly. This process results in the annealling of the single strand oligonucleotides to their homologous counterparts. DNA ligase is added to the mixture to join the now double stranded DNA (dsDNA) segments to form the complete gene.

The ligation reaction is generally characterized by gel electrophoresis in order to assure that a piece of DNA of the expected size (i.e. 400 bp) has resulted. However, due to the complexity of the oligonucleotide mixture in the annealing and ligation steps, obtaining a piece of the correct size does not assure that the new gene has been assembled in the proper order to give a gene with the correct sequence. Therefore, the synthetic gene is cloned into a plasmid for characterization by DNA sequencing. If the sequence is not correct, the gene must be corrected or the entire process must be carried out again. If the gene is of the correct sequence, then it must be subcloned into another plasmid with the proper orientation with respect to gene regulatory signals. The synthetic gene is then used for protein production.

The problem is the above-described production of double-stranded DNA sequences such as genes involve a number of multiple reactions, cloning and isolation steps.

Figure 1 is a schematic illustration of the method of this invention.

Figure 2 shows the relevant genetic components of pKH125.

Figure 3 shows the native and synthetic HIV-I tat gene.

Figure 4 shows oligonucleotide designed to construct the HIV-I tat gene.

Figure 4a shows three separate portions of the HIV-I tat gene with the specially designed ends for inserting sequentially into pKH125.

Figure 5 shows the strategy for constructing tat in pKH125.

Figure 6 shows single-stranded pKH125 forming a heteroduplex with an oligonucleotide representing a portion of HIV-1 tat gene sequence.

The present invention provides a method of synthesizing a defined double-stranded DNA sequence that eliminates many of the shortcomings of prior art methods. The method comprises the steps of:

A) identifying the double-stranded DNA sequence to be synthesized;

B) providing a single-stranded DNA vector comprising i) a nucleotide sequence comprising deoxyuridine ii) a phage origin of replication and iii) a bacterial origin of replication; and iv) a drug resistance gene such as $\beta$-lactamase (amp);

C) synthesizing a first oligonucleotide which has i) 5$'$ and 3$'$ endings; ii) a nucleotide sequence according to a first fragment of one single strand of the double-stranded DNA sequence to be synthesized and iii) additional nucleotide sequences on both the 5$'$ and 3$'$ ends which a) are at least partially complementary to nucleotide sequence of the DNA single-stranded vector and b) the complementarity at the 3$'$ end is sufficient for priming;

D) hybridizing the first oligonucleotide fragment to the single-stranded vector to form a heteroduplex;

E) synthesizing a deoxyuridine free second complementary strand for the single-stranded DNA vector using the 3$'$ end of the first oligonucleotide as a primer thereby forming a double stranded vector;

F) transfecting an E.coli strain which contains the enzyme uracil N-glycosylase (ung$^+$) with the double-stranded DNA vector;

G) selecting from the cloned ung$^+$, drug resistant (for example amp resistant) E.coli colonies double-stranded DNA vectors containing the first oligonucleotide;

H) transfecting a dUTPase and uracil N-glycosylase deficient E.coli strand (dut$^-$, ung$^-$) with the vectors selected in G);

I) isolating single-strand vectors comprising (i) deoxyuridine in the nucleotide sequence and (ii) the first oligonucleotide from the transfected E.coli of H);

J) synthesizing a second oligonucleotide which has

i) 5$'$ and 3$'$ ending;

ii) a nucleotide sequence according to a second fragment of the same single strand of the double-stranded DNA sequence to be synthesized selected in step C)i); and

iii) additional nucleotide sequences on

a) the 5$'$ end that are complementary to nucleotide sequences on the 3$'$ end of the first synthesized oligonucleotide fragment and

b) the 3$'$ end that are the same as the additional sequences on the 3$'$ end of the first synthesized oligonucleotide fragment.

K) repeating steps D) through G) to insert the second oligonucleotide in the vector of I) in frame with the first synthesized oligonucleotide fragment; and

L) repeating step K) and then D) through J) for additional oligonucleotide fragments until synthesis of the entire defined double-stranded DNA sequence identified in step A) has been completed.

The method of the present invention offers the following advantages over conventional methods for constructing double-stranded DNA sequences (dsDNA) such as genes:

- the accuracy of assembly of the correct double-stranded DNA sequence or gene is equivalent to the accuracy of oligonucleotide-directed mutagenesis, which is >99% (Kunkel et al, Method in Enzymology, 154, 367-382, 1987).

- only a few long oligonucleotides need be synthesized and purified, corresponding to only one strand of the dsDNA;

- the sequence of the DNA construction can be checked at each cycle by checking the template to be used in the next construction cycle (from step K);

- at the end of a dsDNA synthesis, a gene does not require subcloning since it can be constructed directly in a plasmid which has the appropriate regulatory sequences for gene expression;

- if an error has occurred during construction, it can be corrected by mutagenesis without subcloning;

- since the dsDNA is in a plasmid with fl origin, a gene can be constructed out of translational frame with the plasmid gene expression system to avoid problems which might arise if the synthetic gene product (protein) were toxic to the host cell, (the gene can later be put into frame by mutagenesis);

- the process is efficient, with each cycle taking about 4 days;

- the size (bases of DNA) of the double-stranded DNA sequence made in each cycle is only limited to the present and future synthetic chemistry limits of automated DNA synthesizers;

- gene products (proteins) can be directly analyzed for expression and activity, since the gene can be synthesized in an expression vector;

- a gene can be synthesized directly as an in-frame gene fusion with a second gene (such as $\beta$-galactoside) already in the plasmid, creating novel chimeric genes;

- a synthetic gene can be directly altered by site-directed mutagenesis without subcloning steps, making protein structure-function studies more convenient to perform;

- parts of genes, or exon regions of genes, or chimeric genes corresponding to single or combinations of functional regions of proteins or domains of proteins, are conveniently synthesized and expressed;

- genes or parts of genes or chimeric genes which code for proteins which could be used for enzymes, biochemicals, pharmaceuticals, vaccines, diagnostic reagents or agricultural reagents could be conveniently synthesized and expressed; and
- synthetic genes or native genes obtained by other methods containing extensive mutations or deletions or insertions can be conveniently repaired by mutagenesis using long oligonucleotides.

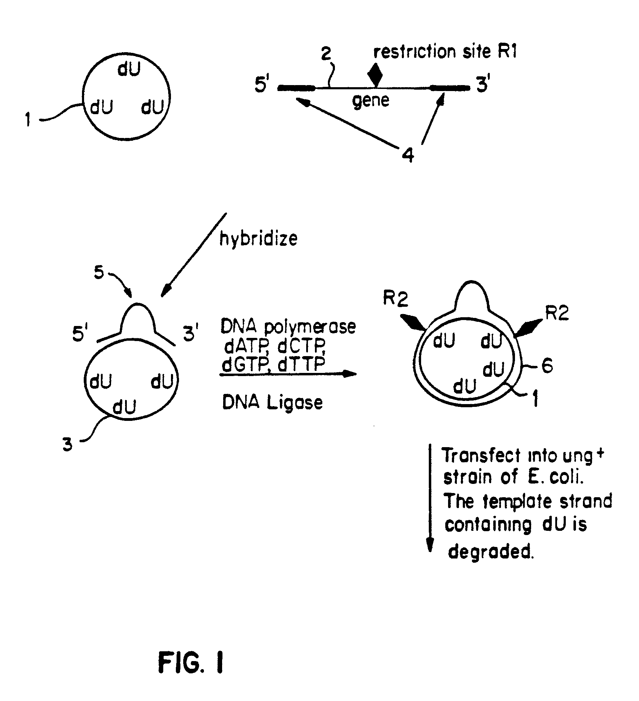

Steps A) through I) of the method are presented schematically in Figure 1. Therein is shown a single-stranded DNA plasmid (ssDNA) template 1 which contains deoxyuridine in place of deoxythymidine in a part of its DNA sequence. The template is derived from a plasmid comprising a phage origin of replication (not shown). Such plasmids are sometimes referred to as phagemids. An example of a phage origin of replication is the fl phage intergenic region [fl(IG)] of bacteriophages. This region contains the origin of replication for the synthesis and packaging of single-stranded DNA.

Methods for producing single-stranded deoxyuridine-containing DNA from these plasmids are well known in the art. In general, such methods involve:

1) providing a plasmid or phagemid having a fl phage intergenic region [fl(IG)] (ss phage origin of replication);

2) transferring the plasmid into an E.coli strain that

i) is deficient in dUTPase and uracil N-glycosylase and

ii) includes a F′ element;

3) infecting E.coli resulting from 2) with a helper phage thereby forming a single-stranded DNA version of the plasmid of 1) containing a variable percent of uracil bases (ssP); and

4) isolating single-stranded plasmid (ssP) containing uracil produced by the infected E.coli.

Examples of useful plasmids include pKH125, pUCF1 (former Pharmacia) Genescribe. pTZ18R, pTZ18BU, pTZ19U (U.S. Biochemical) and pVT100, 101, 102 and 103 (Vernet and Thomas).

In Figure 1 there is also shown an oligonucleotide 2 containing a portion of one strand of a defined DNA sequence. The oligonucleotide 2 comprises a unique restriction site R1 and additional DNA sequences 4 at the 5′ and 3′ ends that are complementary to a portion of the DNA sequence in the ssDNA template 1. The oligonucleotide 2 is hybridized (step D) to the ssDNA template 1. The result is shown in 3. The complementary regions 4 of the oligonucleotide 2 are hybridized to the ssDNA template. The noncomplementary portions of the oligonucleotide 5 form a loop that does not hybridize to the ssDNA template 1.

The 3′ end of the oligonucleotide serves as a primer for synthesizing (step E) a second strand 6 of DNA that is complementary to the ssDNA template 1. The complementary strand synthesis is carried out conventionally in the presence of dATP, dGTP, dTTP and dCTP, DNA polymerase (Klenow fragment) and DNA ligase as shown schematically in Figure 1. The result of the synthesis is a dsDNA sequence 6 with restriction sites R2 bracketing the inserted oligonucleotide for screening purposes. This dsDNA 6 with the oligonucleotide 2 is transfected into an ung+ strain of E.coli in which the strand comprising the deoxyuridine 1 is degraded. The DNA strand containing the inserted oligonucleotide 2 is maintained and replicated by E.coli is isolated and then found by restriction enzyme cleavage (R1 and/or R2) and DNA sequencing. This new ssDNA template is prepared from the corrected clones for the next cycle of DNA synthesis according to the method of this invention.

The foregoing description involves one cycle of the method. A second, third and fourth cycle or as many cycles as needed are carried out to complete the synthesis of the defined double-stranded DNA sequence.

After each cycle, clones can be isolated and plasmids checked for the inserted oligonucleotide fragment by cutting with restriction enzyme at R1. R1 is unique to the inserted fragment. In addition, the size of the inserted fragment can be checked by cutting with restriction enzymes at positions R2. R2 flanks both sides of the inserted oligonucleotide fragment. For each cycle the length of the R2 restriction fragment increases exactly by the length of the inserted fragment.

The present method combines, in novel and unobvious ways, insertion of related oligonucleotides by site-directed mutagenesis and genetic enrichments to construct a defined double-stranded DNA sequences (dsDNA), such as genes, in E.coli. Construction of the HIV-I tat gene is used as an example of this new method. The method is generally applicable to the synthesis of any dsDNA sequence.

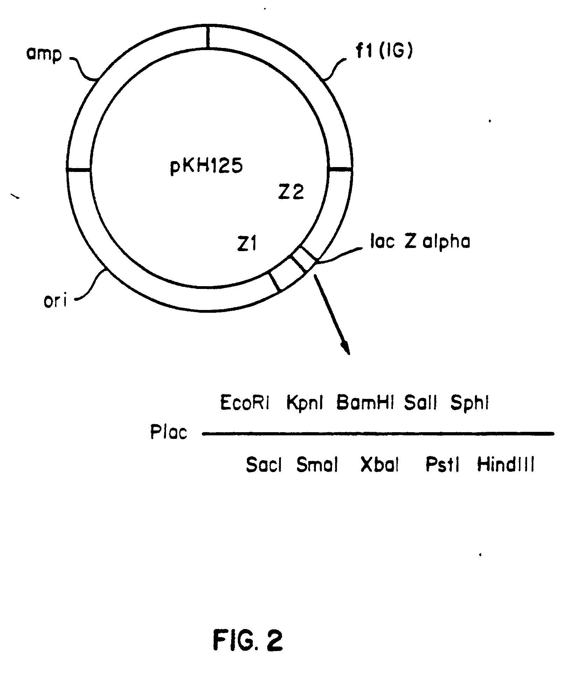

The method, as illustrated in the following example results in direct synthesis of a gene on a plasmid containing the proper regulatory sequences for direct gene and protein expression. This eliminates the need to subclone the completed gene into an expression vector. Plasmid pKH125 (Figure 2) is used to illustrate the method. It contains a phage origin of replication [fl(IG)]; an antibiotic selection marker (amp), and a bacterial origin of replication (ori). The plasmid also contains a multiple cloning site (mcs) within a lacZ alpha DNA sequence. This plasmid was made by one of the coinventors herein. However, any plasmid containing a ssDNA phage origin of replication will be useful in this method.

In insertional gene synthesis [steps A) through G) of Figure 1] large oligonucleotides comprising a single strand of the defined dsDNA sequence, or defined fragments thereof surrounded by short sequences designed to base pair to a region on a single-stranded template, are hybridized stepwise on the template, in vitro.

The recovery of plasmids containing inserted oligonucleotides is greatly simplified by steps B), E) and F) which enrich for mutants. E.coli strains (dut⁻ ung⁻) with mutations in dUTPase and uracil N-glycosylase are used to propagate the ssDNA templates of step B) to which the oligonucleotide fragments are hybridized in step D). The result is the production of ssDNA templates that contains deoxyuridine substituted for deoxythymidine at random positions in the DNA sequence. The complementary strand to this template is synthesized in vitro using an oligonucleotide as the primer, and the four conventional DNA nucleotides (dATP, dCTP, dGTP and dTTP).

Transfection of plasmids made by this procedure into E.coli containing uracil N-glycosylase (ung⁺) results in selective degradation of the plasmid strand containing deoxyuridine. This process greatly enriches for the dsDNA plasmids containing the oligonucleotide sequence made in vitro with the conventional nucleotides. The frequency of recovering mutant plasmids using this genetic enrichment is approximately ≧30%.

However, insertion of short sequences into plasmids by mutagenesis would require many rounds of mutagenesis to construct an entire gene. For convenience, oligonucleotides are made approaching 140 bases. This is about the current limit of Auto-DNA synthesizers. Assuming about 20 bp of homology with template sequences are needed on each end for proper hybridization with the template, 100 nucleotides can be inserted in each cycle of the method [steps A) through G)]. Four cycles would result in synthesis of a 400 bp dsDNA sequence which could encode a protein of approximately 15,000 mw. Since each cycle takes approximately 4 days, the complete synthesis, characterization and expression of such a gene takes less than 1 month. Most current procedures require in excess of 1 month to synthesize and characterize a gene of comparable size.

It will be understood by those skilled in the art that the method is applicable to almost any length of gene. As the ability to make larger oligonucleotides expands, this method's applicability will similarly expand.

This synthesis of the oligonucleotides are carried out using conventional automated methods.

The laboratory techniques, including the chemicals and apparatus, required to carry out the method of this invention are conventional and are described in Maniatis et al and also in Kunkel et al (both cited hereinbefore).

The following example illustrates the usefulness of this method.

Example - Synthesis of a HIV-I tat Gene

I. Design of a Synthetic tat Gene

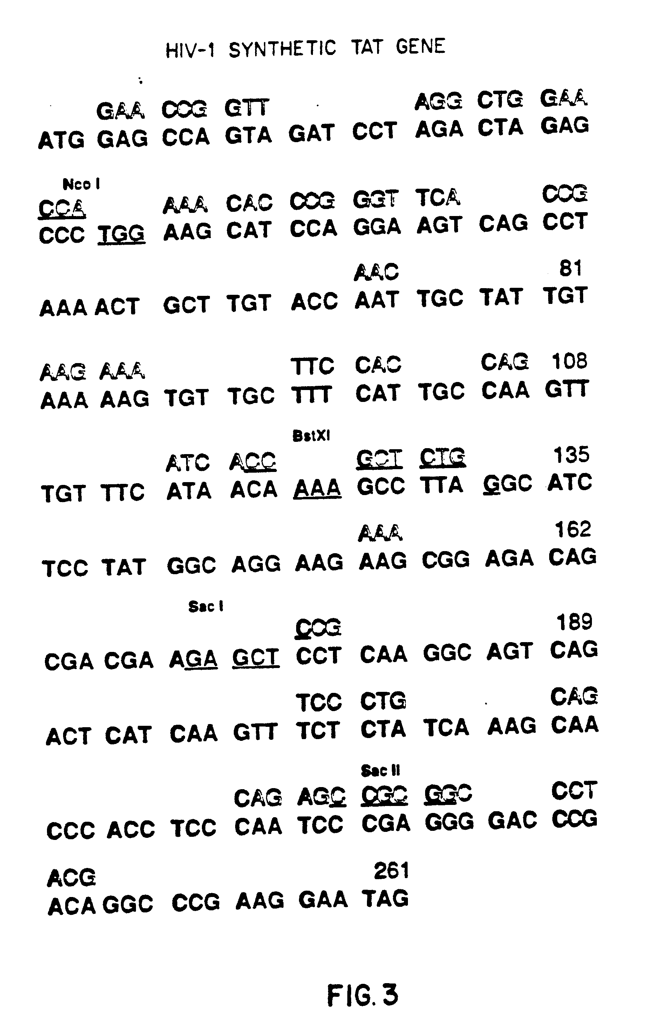

A synthetic tat gene was designed by converting native codons of HIV-I isolate BH10, shown in bold type in Figure 3, to alternative codons, shown in open type above the native codons in Figure 3, using the known degeneracy of the genetic code. A total of 33 out of the gene's 87 codons were redesigned, resulting in a synthetic gene which is 62% homologous in nucleotide sequence to the native gene. The redesigned codons in the synthetic gene were made to match the preferred E.coli codons for increased gene expression. In addition, some codons formed new restriction sites (Ncol, BstXI, SacI and Sac II in Figure 3) that are diagnostic for the gene. These sites are also unique in pKH125 which is used in this example. These sites were designed into the synthetic tat gene for the purpose of screening clones following each cycle of the method of this invention. The synthetic tat gene sequence, when translated using the genetic code, results in the same protein sequence derived from the native gene.

II. Construction of the Synthetic tat Gene

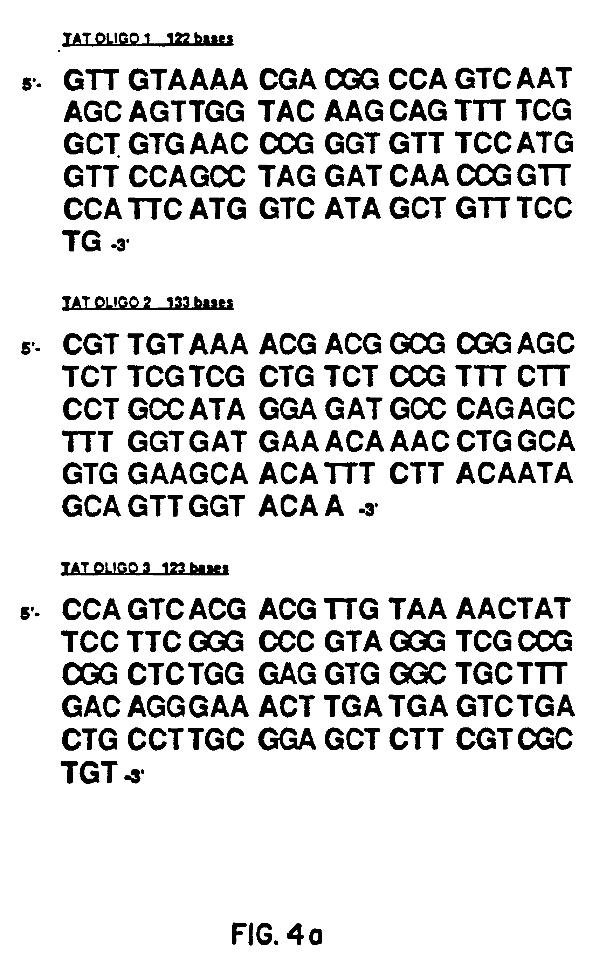

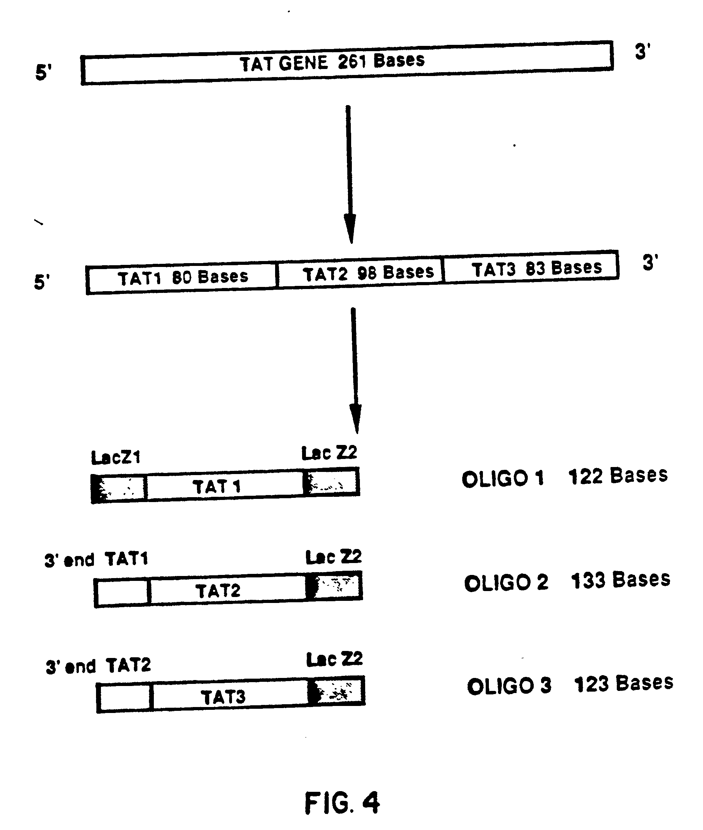

The complete scheme for construction of oligonucleotides (oligo) to make the synthetic tat gene by insertional mutagenesis is given on Figure 4. The 261 BP synthetic tat gene sequence, shown in Figure 3, was divided for oligonucleotide synthesis into 3 fragments tat 1, tat 2 and tat 3 shown in Figure 4. The DNA sequences of tat1, tat2 and tat3 are shown in Figure 4a.

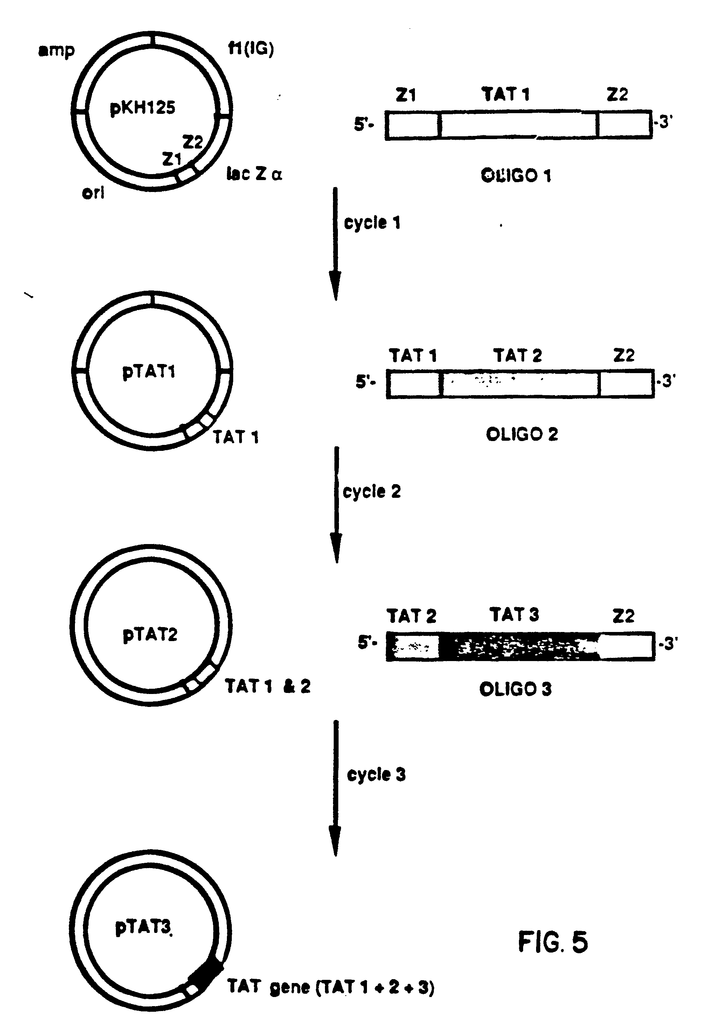

The strategy used for the design of the oligonucleotide fragments for the construction of the synthetic tat gene is shown in Figure 5. An ollgonucleotide fragment (oligo 1 in Figures 4 and 5) was synthesized which comprised the first 1/3 of the DNA sequence of one strand of the tat gene (tat 1) flanked on its 5′ end by 20 bases from the lacZα fragment gene (Z1) in pKH125 (Figures 3, 4 and 5) and on its 3′ end by another 20 bases from the lacZα fragment gene (Z2) in pKH125 (Figures 3, 4 and 5).

Oligo 1 and all subsequent oligo fragments were synthesized with conventional automated techniques. The lacZα gene in pKH125 codes for the α fragment of E.coli β-galactosidase contained on plasmid pKH125 (Figure 2).

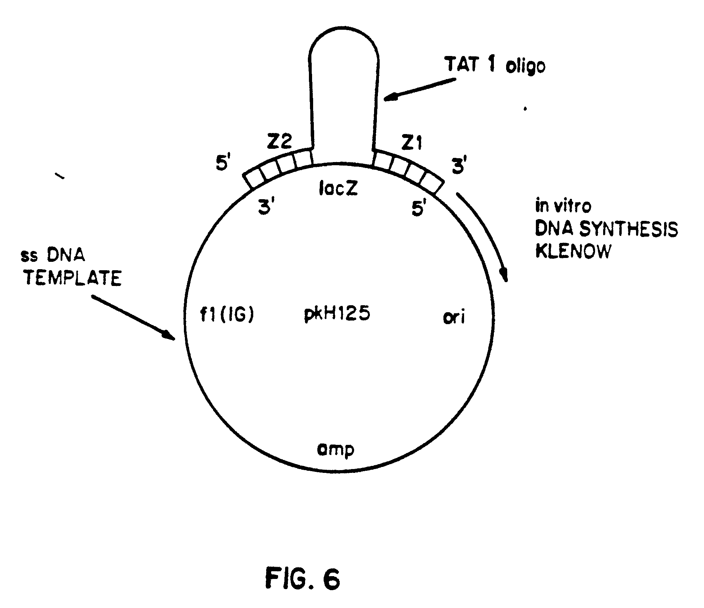

This plasmid also comprises fl(IG), an ampicillin marker (AMP); an E.coli origin of replication (ORI) and a multiple cloning site (MCS). The parts of oligo 1 designated Z1 and Z2 (Figures 3 and 4) were designed to be complementary to regions within the lacZα gene and thus are able to hybridize directly to these regions on single-stranded pKH125 (Figure 6). The part of oligo 1 designated tat 1 comprises the first 1/3 of one strand of the synthetic tat gene and is not complementary to any region of pKH125. Thus, upon mixing of single-stranded pKH125 and Oligo 1, the tat 1 region forms a large loop anchored by Z1 and Z2 (Figures 1 and 6).

Plasmid pKH125 was prepared in a single-stranded form (referred to as the template strand) and contained deoxyuridine in place of deoxythymidine as discussed previously herein. oiigo 1 was hybridized to ss-pKH125 using conventional recombinant DNA procedures. The complementary strand to this plasmid was synthesized in vitro using standard deoxynucleotides (no dUTP) with the Klenow fragment of DNA polymerase (Figures 1 and 6). The resulting plasmid contained deoxyuridine in the template strand (Figure 1) and tat 1 sequence on the complementary strand. When this double stranded (ds) plasmid was transfected into E.coli strain TBI, which contains the enzyme uracil N-glycosylase (ung$^+$), the deoxyuridine containing template strand was selectively degraded by the host E.coli. This resulted in enrichment of the complementary strand containing tat 1.

E.coli tat 1 clones were selected as white colonies on LB plates containing ampicillin and XGAL. Several colonies were checked for the presence of a plasmid with the tat 1 insert by restriction analysis and gel electrophoresis as follows. Plasmids from the colonies were isolated as minipreps according to conventional DNA cloning procedures and then digested with the restriction enzyme Ncol. The presence of an Ncol site (R1 on Figures 1 and 3) in the plasmid is diagnostic of the tat 1 insert in pKH125. Plasmids from six clones were linearized by Ncol, and these were further characterized by DNA sequencing, which confirmed that tat 1 was inserted into pKH125 within the lacZα fragment.

One of these tat 1 positive plasmids was designated as ptat1 resulting from pKH125 + tat 1. It was used to prepare a single-stranded template for the second cycle of gene synthesis with oligo 2 (tat 2).

A second oligonucleotide fragment (oligo 2 in Figures 4 and 5) was synthesized which comprised the second 1/3 of the DNA sequence of the strand of tat gene (tat 2) from which oligo 1 was prepared. Oligo 2 was flanked on its 5′ end by the last 20 bases from the 3′ end of tat 1, and on its 3′ end by 20 bases from Z2. Thus, oligo 2 was designed so that its tat 2 region would form a contiguous sequence with tat 1 when it was inserted into ptat1.

Oligo 2 was hybridized to single-stranded ptatl as previously described for Oligo 1. The complementary strand for ptat1 was synthesized in vitro as described previously. Following transfection, clones were selected and plasmids prepared as minipreps as before. Clones were digested with the restriction enzyme BstXl (Figure 3 and R1 in Figure 1), to check for the tat 2 insert, which contains a BstBXl restriction site. Plasmids from selected clones were designated a, b and c and linearized with BstXl. In addition, plasmids were digested with Pvull (R2 in Figure 1). Plasmids from clones a and c yielded a 0.46 kb Pvull fragment, while the plasmid from clone b gave a fragment of 0.38 kb. The larger Pvull fragment of clones a and c was due to the presence of the tat 2 insert. The presence of the tat 2 insert in plasmids from clones a and c was confirmed by DNA sequencing.

A plasmid from one of these correct clones (α) was designated as ptat2 and used to prepare the ssDNA template for the third and last cycle of gene synthesis. A third oligonucleotide (oligo 3, Figures 4 and 5) was then synthesized containing approximately the last 1/3 of the DNA sequence of the strand of tat gene (tat 3) from which oligos 1 and 2 were prepared. Oligo 3 was flanked on its 5′ end by 20 bases from the 3′ end of tat 2 and on its 3′ end by 20 bases from Z2. A third cycle of gene synthesis was carried out. Plasmids from 10 clones were checked for the tat 3 insert. Two of the plasmids were linearized by digestion with Sac II (R1 in Flgure 1). In addition, these two plasmids were digested with Puvll (R2 in Figure 1). These plasmids yielded a larger Puvll fragment (0.54 kb vs. 0.46 kb). The presence of the tat 3 insert which now completed the full length tat gene, was confirmed by DNA sequencing.

## Claims

1. A method of synthesizing a defined double-stranded DNA sequence comprising the steps of:

A) identifying the double-stranded DNA sequence to be synthesized;

B) providing a single-stranded DNA vector comprising i) a nucleotide sequence comprising deoxyuridine and ii) a phage origin of replication and iii) a bacterial origin of replication; iv) a drug resistance gene;

C) synthesizing a first oligonucleotide which has i) 5′ and 3′ endings; ii) a nucleotide sequence according to a first fragment of one single strand of the double-stranded DNA sequence to be synthesized and iii) additional nucleotide sequences on both the 5′ and 3′ ends which a) are at least partially complementary to nucleotide sequence of the DNA single-stranded vector and b) the complementarity at the 3′ end is sufficient for priming;

D) hybridizing the first oligonucleotide fragment to the single-stranded vector to form a heteroduplex;

E) synthesizing a deoxyuridine free second complementary strand for the single-stranded DNA vector using the 3′ end of the first oligonucleotide as a primer thereby forming a double stranded vector;

F) transfecting an E.coli strain which contains the enzyme uracil N-glycosylase (ung⁺) with the double-stranded DNA vector;

G) selecting from the cloned ung⁺, drug resistant E.coli colonies double-stranded DNA vectors containing the first oligonucleotide;

H) transfecting a dUTPase and uracil N-glycosylase deficient E.coli strand (dut⁻, ung⁻) with the vectors selected in G);

I) isolating single-strand vectors comprising (i) deoxyuridine in the nucleotide sequence and (ii) the first oligonucleotide from the transfected E.coli of H);

J) synthesizing a second oligonucleotide which has

i) 5′ and 3′ ending;

ii) a nucleotide sequence according to a second fragment of the same single strand of the double-stranded DNA sequence to be synthesized selected in step C)i); and

iii) additional nucleotide sequences on

a) the 5′ end that are complementary to nucleotide sequences on the 3′ end of the first synthesized oligonucleotide fragment and

b) the 3′ end that are the same as the additional sequences on the 3′ end of the first synthesized oligonucleotide fragment.

K) repeating steps D) through G) to insert the second oligonucleotide in the vector of I) in frame with the first synthesized oligonucleotide fragment; and

L) repeating step K) and then D) through J) for additional oligonucleotide fragments until synthesis of the entire defined double-stranded DNA sequence identified in step A) has been completed.

2. The method of claim 1 wherein the defined double-stranded DNA sequence to be synthesized comprises a gene.

3. The method of claim 2 wherein the single-strand vectors into which the oligonucleotide fragments are inserted contains all of the DNA regulatory sequences for gene and protein expression.

4. The method of claim 2 or 3 wherein the oligonucleotide fragments include unique restriction sites.

5. The method of claim 2 or 3 wherein the oligonucleotide fragments have gene codons that have been optimized for gene and protein expression in E.coli.

6. The method of claim 1 wherein the presence of an inserted oligonucleotide fragment in the single-strand vector of step I) is confirmed by restriction analysis, DNA sequencing or both.

7. The method of claim 6 wherein any error in DNA sequence is corrected by site-directed mutagenesis.

8. A synthetic tat gene from HIV-I having the DNA sequence according to Figure 3.

FIG. I

amp

f1 (IG)

pKH125

Z2

Z1

lac Z alpha

ori

Plac

EcoRI  KpnI  BamHI  SalI  SphI

SacI  SmaI  XbaI  PstI  HindIII

FIG. 2

## HIV-1 SYNTHETIC TAT GENE

```
           GAA  CCG  GTT     .              AGG  CTG  GAA
      ATG  GAG  CCA  GTA  GAT  CCT  AGA  CTA  GAG

       Nco I
      CCA        AAA  CAC  CCG  GGT  TCA        CCG
      CCC  TGG  AAG  CAT  CCA  GGA  AGT  CAG  CCT

                                  AAC              81
      AAA ACT  GCT  TGT  ACC  AAT  TGC  TAT  TGT

      AAG AAA              TTC  CAC        CAG  108
      AAA  AAG  TGT  TGC  TTT  CAT  TGC  CAA  GTT

                              BstXI
           ATC  ACC        GCT  CTG        135
      TGT  TTC  ATA  ACA  AAA  GCC  TTA  GGC  ATC

                                  AAA             162
      TCC  TAT  GGC  AGG  AAG  AAG  CGG  AGA  CAG

           Sac I
                         CCG                      189
      CGA  CGA  AGA  GCT  CCT  CAA  GGC  AGT  CAG

                         TCC  CTG        .   CAG
      ACT  CAT  CAA  GTT  TCT  CTA  TCA  AAG  CAA

                              Sac II
                    CAG  AGC  CGC  GGC        CCT
      CCC  ACC  TCC  CAA  TCC  CGA  GGG  GAC  CCG

      ACG                   261
      ACA  GGC  CCG  AAG  GAA  TAG
```

## FIG. 3

TAT OLIGO 1   122 bases

5'-  GTT GTA AAA CGA CGG CCA GTC AAT
AGC AGT TGG TAC AAG CAG TTT TCG
GCT GTG AAC CCG GGT GTT TCC ATG
GTT CCA GCC TAG GAT CAA CCG GTT
CCA TTC ATG GTC ATA GCT GTT TCC
TG -3'

TAT OLIGO 2   133 bases

5'-  CGT TGT AAA ACG ACG GCG CGG AGC
TCT TCG TCG CTG TCT CCG TTT CTT
CCT GCC ATA GGA GAT GCC CAG AGC
TTT GGT GAT GAA ACA AAC CTG GCA
GTG GAA GCA ACA TTT CTT ACA ATA
GCA GTT GGT ACA A -3'

TAT OLIGO 3   123 bases

5'-  CCA GTC ACG ACG TTG TAA AAC TAT
TCC TTC GGG CCC GTA GGG TCG CCG
CGG CTC TGG GAG GTG GGC TGC TTT
GAC AGG GAA ACT TGA TGA GTC TGA
CTG CCT TGC GGA GCT CTT CGT CGC
TGT -3'

FIG. 4a

FIG. 4

FIG. 5

FIG. 6